(19) Europäisches Patentamt
European Patent Office
Office européen des brevets

(11) Publication number: 0 050 799
B1

(12) # EUROPEAN PATENT SPECIFICATION

(45) Date of publication of patent specification: 19.12.84

(51) Int. Cl.$^3$: C 07 C 101/20, C 07 C 99/00, C 07 C 125/065, C 09 B 19/00

(21) Application number: 81108343.5

(22) Date of filing: 15.10.81

(54) **New processes for the production of di-alkyl esters and optically active mono-alkyl esters of 3-aminoglutaric acid.**

(30) Priority: 21.10.80 JP 146343/80
21.10.80 JP 146344/80
21.10.80 JP 146345/80

(43) Date of publication of application:
05.05.82 Bulletin 82/18

(45) Publication of the grant of the patent:
19.12.84 Bulletin 84/51

(84) Designated Contracting States:
AT BE CH DE FR GB IT LI NL

(56) References cited:
FR-A-2 408 579
US-A-4 205 183
J. ORG. CHEM., vol. 27, July 1962, pages 2466-2470 Washington D.C., U.S.A. A.J. JOSEY et al.: "M-Functionally substituted pyrroles"
IL FARMACO - ED. SC., vol. 33, April 1978, pages 237-252 Pavia J. GILBERT et al.: "Analogues azotes de l'emetine: synthèse et évaluation de l'activité antiamibienne et antitumorale de l'aza-3 emetine, et essais d'obtention de l'aza-2 emetine"
J.AM. CHEM. SOC., vol. 93, June 1971, pages 2897-2904 Washington D.C., U.S.A. R.F. BORCH et al.: "The cyanohydridoborate anion as a selective reducing agent"

(73) Proprietor: ZAIDAN HOJIN BISEIBUTSU KAGAKU KENKYU KAI
14-23, Kami Osaki 3-chome
Shinagawa-ku Tokyo (JP)

(72) Inventor: Ohno, Masaji
1565-13, Koshigoe
Kamakura-shi Kanagawa-ken (JP)
Inventor: Umezawa, Hamao
23, Toyotama Kita 4-chome Nerima-ku
Tokyo (JP)

(74) Representative: Reuter, Johann-Heinrich, Dr. et al
HOECHST AKTIENGESELLSCHAFT Zentrale Patentabteilung Postfach 80 03 20
D-6230 Frankfurt/Main 80 (DE)

(56) References cited:
NATURE, vol. 186, no. 4718, 2nd April 1960, pages 75-76 Basingstoke, U.S.A. S.G. COHEN et al.: "Requirements for stereospecificity in hydrolysis by chymotrypsin: Diethyl Beta-aceta-midoglutarate"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 050 799 B1

Courier Press, Leamington Spa, England.

**Description**

BACKGROUND OF THE INVENTION

*Field of the Invention*

This invention is relates to processes for the production of optically active (S)-mono-alkyl ester and (R)-mono-alkyl ester of 3-aminoglutaric acid starting from the optically inactive di-alkyl ester of 3-aminoglutaric acid by treatment with esterase for preferential, hydrolytic removal of one ester-forming alkyl group.

*Description of the Prior Art*

3-Aminoglutaric acid is one of known β-amino acids and is a di-basic amino acid which is important as the intermediate for the preparation of a variety of physiologically active substances. Such physiologically active substances include negamycin and a series of carbapenem β-lactam type antibiotics, typically thienamycin, which are known to be highly active against Gram-negative bacteria. These physiologically active substances are known as antibiotics having high antibacterial activity against various bacteria and have been produced in extremely low yield principally by fermentation. In this situation, there is a great demand for a highly efficient process of producing these physiologically active substances synthetically.

It is well known that only one enantiomer having desired absolute configuration is physiologically active, but the antipode is inactive. As representative examples thereof, there may be mentioned penicillins, cephalosporins and thienamycin. For instance, thienamycin is a compound containing three asymmetric carbons and represented by the following absolute structure:—

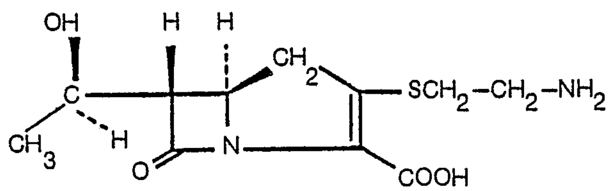

Thienamycin may be synthesized via (S)-4-alkoxycarbonylmethylazetidine-2-on of the formula

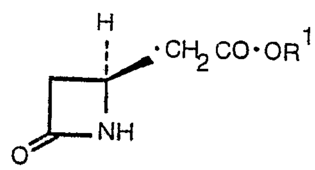

wherein R¹ denotes an alkyl group, which prepared by multiple steps starting from a di-alkyl ester of L-aspartic acid of the formula

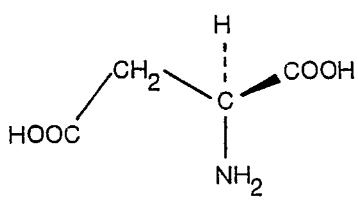

(see Japanese patent application pre-publication "Kokai" No. 27169/80 and U.S. patent application SN. 933323 filed August 14, 1978).

We have expected that if an optically active β-(S)-mono-alkyl (half)ester of 3-aminoglutaric acid is produced synthetically, it will be useful as an intermediate for the production of the above-mentioned azetidinone derivative having (S)-configuration which is, in turn, essential as a starting material for the synthesis of thienamycin and hence generally as a starting material for the synthesis of carbapenem antibiotics. With this expectation, we have proceeded our researches to find out that when an optically inactive β-benzyloxycarbonylaminoglutaric acid di-alkyl ester which may be prepared synthetically from the aforesaid 3-aminoglutaric acid di-alkyl ester is treated with an ester-hydrolyzing enzyme (pig liver esterase), one ester-forming alkyl group can be removed preferentially by hydrolysis without hydrolytic removal of the other ester-forming alkyl group, whereby an optically active monoalkyl ester (half ester) is yielded. When this optically active mono-ester is subjected to a conventional N-deprotected method for removal of the amino-protecting benzyloxycarbonyl group, there is afforded an optically active β-(S)-aminoglutaric acid mono-alkyl ester. We have further found that when the optically inactive 3-aminoglutaric acid di-alkyl ester is immediately treated with the ester-hydrolysing enzyme (pig liver esterase), one ester-forming alkyl group is removed preferentially by the hydrolysis without cleavage of the other ester-forming alkyl group, affording an optically active β-(R)-aminoglutaric acid mono-alkyl ester.

An object of this invention is to provide a new and efficient process for the production of prochiral 3-aminoglutaric acid di-alkyl ester. Another object of this invention is to provide processes for the production of optically active β-(S)-aminoglutaric acid monoalkyl ester and optically active β-(R)-aminoglutaric acid mono-alkyl ester starting from the prochiral 3-aminoglutaric acid d-alkyl ester.

## DETAILED DESCRIPTION OF THE INVENTION

According to a first aspect of this invention, there is provided a process for the production of an optically active 3-(S)-aminoglutaric acid mono-alkyl ester of the formula

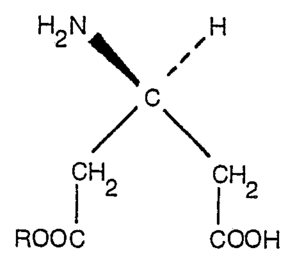

wherein R is an alkyl group of 1 to 6 carbon atoms, which comprises the steps of:—

reacting a β-N-protected aminoglutaric acid di-alkyl ester of the formula

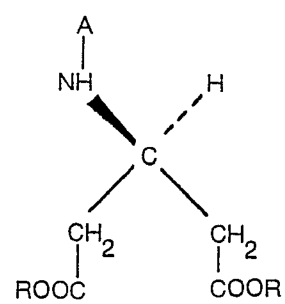

(V)

wherein R is as defined above and A is an amino-protecting group removable by hydrogenolysis of mild hydrolysis, with an ester-hydrolyzing enzyme in an aqueous organic solvent at a pH and at a temperature at which the enzyme employed is enzymatically active, to remove hydrolytically and preferentially one ester-forming alkyl group, R, from the β-N-protected aminoglutaric acid di-alkyl ester without cleavage of the other ester-forming alkyl group,

and then removing the amino-protecting group, A, from the resulting hydrolysis product in a known manner.

The process according to the first aspect invention where β-N-benzyloxycarbonylaminoglutaric acid dimethyl ester is employed as the starting material may schematically be described by the following equation:—

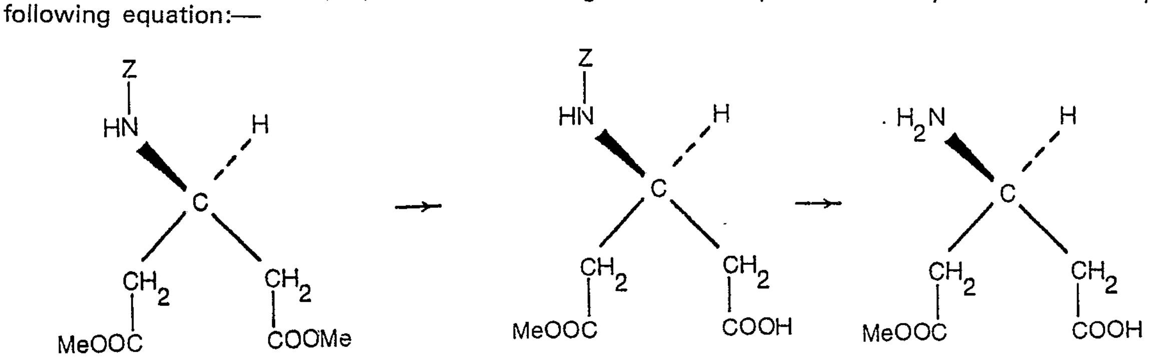

(V') (VI) (IV')

wherein Z denotes benzyloxycarbonyl group

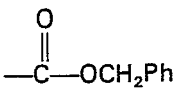

and Me denotes methyl group.

In the reaction shown by the above equation, it is seen that the $\beta$-benzyloxycarbonylaminoglutaric acid dimethyl ester (V′) is a prochiral compound which belongs to the compound of the "Cs-point" class having the symmetrical plane in the molecule, that this compound contains two methoxycarbonylmethyl groups which are positioning with each other in the enantiotropic relationship in the molecule, and that only one of the two methoxycarbonylmethyl groups preferentially undergoes the enzymatic hydrolysis and bring about the removal of one of the ester-forming methyl groups therefrom, affording the optically active $\beta$-(S)-benzyloxycarbonylaminoglutaric acid mono-methyl ester (VI). Subsequent N-deprotection of the compound (VI) yields the optically active $\beta$-(S)-aminoglutaric acid monomethyl ester (IV′).

The stereochemical feature of the reaction equation as shown above is that the starting "prochiral" compound (V′) is converted via the intermediate "chiral" compound (VI) into the final compound (IV′) with the desired stereochemistry.

Various researches which were made in this field of $\beta$-lactam chemistry in the past are limited to ones of academic point of view and are far away from the practical or efficient production of any commercially or therapeutically useful substances such as the antibiotics. Thus, during the research of a field of enzyme chemistry, Cohen and Khedouri reported their discovery that when $\beta$-acetylaminoglutaric acid diethyl ester is hydrolytically treated with $\beta$-chymotrypsin, one of pancreas proteases, this optically inactive di-alkyl ester is converted into optically active $\beta$-(R)-acetylaminoglutaric acid monoethyl ester (S.G. Cohen and E. Khedouri; Nature *186*, 75 (1960)). However, the starting $\beta$-acetylaminoglutaric acid diethyl ester employed in the research of Cohen et al has been acetylated at the amino group thereof, and owing to this, it is very difficult or impossible to convert the resulting optically active hydrolysis product (i.e. $\beta$-(R)-acetylaminoglutaric acid mono-ethyl ester) into a corresponding deacetylated optically active product because the acetylamino group can hardly be converted into the free amino group without causing hydrolytic breakdown of the ester group of said optically active hydrolysis product. Besides, the enzymatic hydrolysis of said starting diethyl ester needed a great amount of $\alpha$-chymotrypsin or a long reaction time, as the rate of the enzymatic hydrolysis involved was very low. For these reasons, it was not considered that the research results by Cohen et al could be further developed so as to provide any method of producing physiologically active substances from the optically active $\beta$-(R)-acetylaminoglutaric acid monoethyl ester, even if certain known procedures of present organic chemistry would be associated therewith in any ingenious way. Furthermore, the optically active $\beta$-(R)-acetylaminoglutaric acid mono-ethyl ester so produced has the (R)-configuration and is not utilisable as a starting material for the preparation of physiologically active substances which should have (S)-configuration at the asymmetric carbon.

As a result of our extensive studies, we have now found that optically active $\beta$-aminoglutaric acid mono-alkyl ester having the (S)-configuration can be produced even using as the substrate compound the 3-aminoglutaric acid di-alkyl ester which was previously considered to have the above-mentioned disadvantageous behaviour in the enzymatic hydrolysis thereof, if the natures of the amino-protecting group as well as the nature of the hydrolysing enzyme employed are selected appropriately in accordance with this invention. Accordingly, we have succeeded to reach the new process of the first aspect of this invention as described hereinbefore.

The $\beta$-protected aminoglutaric acid di-alkyl ester (V) which is employed in the process of the first aspect invention may be prepared by introducing an appropriate known amino-protecting group in a known manner into the 3-aminoglutaric acid di-alkyl ester. The amino-protecting group (A) which is incorporated into the starting di-alkyl ester (V) employed in the process of the first aspect of this invention should be removable by catalytic hydrogenolysis and may preferably be benzyl group, benzhydryl group or an aralkyloxycarbonyl group such as benzyloxycarbonyl. To this end, an alkyloxycarbonyl group of 2 to 7 carbon atoms, such as tert-butoxycarbonyl which is removable by mild hydrolysis eg. with trifluoroacetic acid may be used. The introduction of these amino-protecting groups may be achieved by conventional amino-protecting technique known in the synthesis of peptides. The ester-forming alkyl group (R) in the starting di-alkyl ester (V) is a lower alkyl group of 1 to 4 carbon atoms, such as methyl and ethyl.

The ester-hydrolyzing enzyme (esterase) employed in the present process is chosen from pig liver.

In the process according to the first aspect of this invention, the enzymatic reaction may conventiently be carried out in such a manner that the starting $\beta$-protected aminoglutaric acid di-alkyl ester (V) is dissolved in a volume of a phosphate buffer solution and the substrate solution so obtained is admixed with the esterase, followed by stirring at ambient temperature to allow the reaction to proceed. The reaction medium may usually be water but, if necessary, be water containing therein a water-miscible organic solvent such as dimethylformamide, acetone or a lower alkanol, for example, methanol at a concentration of not more than 10% or at a such a concentration that the activity of the esterase is degraded by the presence of the organic solvent. The reaction medium is preferable to have

a pH of 7.0 to 9.0 and specially a pH of 7.0 to 8.0. The reaction temperature may usually be in a range of 20 to 30°C, although the enzymatic reaction may proceed at any temperature at which the enzyme employed is remaining active. The reaction time required is usually in a range of 1 to 2 hours, though it depends on the concentrations of the starting compound and the enzyme employed. The amount of the enzyme used may be catalytic but may preferably be in a range of 0.001 to 1% by weight (as protein) based on the starting substrate compound (V) in order to obtain a favorably high rate of the reaction.

After the enzymatic reaction is completed, the reaction solution is adjusted to pH of not higher than 7.0 and then extracted with (in examples 1—3 also with methylene chloride). The methanolic extract is concentrated to afford a crude product as oil, which may be isolated and purified by chromatography on silica gel column developed with ethyl ether or methylene chloride. In this way, the desired β-(S)-protected aminoglutaric acid mono-alkyl ester is afforded in a high yield. This N-protected intermediate product is subjected to removal of the amino-protecting group (A) according to a conventional N-deprotection technique such as catalytic hydrogenolysis or mild hydrolysis with trifluoroacetic acid, whereby the desired product, β-(S) aminoglutaric acid mono-alkyl ester (IV) is yielded. This final product shows an optical purity which varies depending on the reaction conditions employed but is of a negative $[\alpha]_D$ value. The absolute stereochemistry of this product was determined to be S-configuration by cyclising this product into a β-lactam compound and then comparing the optical rotation of the latter with that of an authentic sample prepared by a known route. Thus, with the process of the first aspect of this invention, it is observed that only one of the two enantropic alkoxycarbonylmethyl groups present in the starting substrate compound (V) can preferentially undergo the hydrolytic removal of the alkyl group of the "pro-S" nature therefrom, giving the β-(S)-protected aminoglutaric acid monoalkyl ester. This is surprising, as it is unexpectable from the fact that the opposite configuration (R) was shown by the hydrolysis product which was obtained when substrate 3-aminoglutaric acid di-alkyl ester having free amino group or acetylated amino group was enzymatically hydrolysed by the prior art method in the same manner as in the process of the first aspect of this invention.

β-(S)-Aminoglutaric acid mono-methyl ester and mono-ethyl ester as obtained by this first aspect invention are new compounds which exhibit antibacterial activity against Gram-positive bacteria, particularly against *Staphylococcus aureus* and hence are useful as antibacterial agent. Furthermore, the optically active β-(S)-aminoglutaric acid mono-alkyl ester as obtained by this invention can readily be converted by one reaction step into a 4-alkoxycarbonylmethylazetidine-2-on which is an important intermediate for the synthesis of carbapenem β-lactam antibiotics, when it is processed by a method of organic chemistry comprising reacting it with triphenylphosphine and 2,2'-dipyridyl disulfide in acetonitrile (see our co-pending Japan patent applications Nos. 146343/80, 146344/80, 146345/80).

According to a second aspect of this invention, there is provided a process for the production of an optically active β-(R)-aminoglutaric acid mono-alkyl ester of the formula

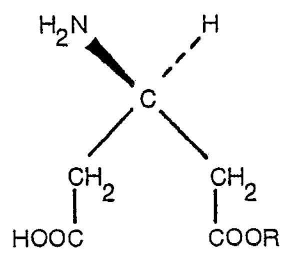

(VII)

wherein R is an alkyl group of 1 to 6 carbon atoms, which comprises the step of:—

reacting a β-aminoglutaric acid di-alkyl ester of the formula

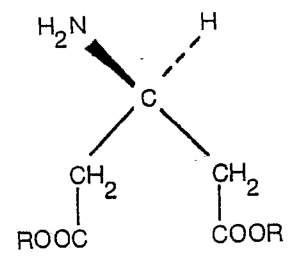

(VIII)

wherein R is as defined above, with an ester/hydrolyzing enzyme in an organic solvent at a pH and at a temperature at which the enzyme employed is enzymatically active, to remove hydrolytically and preferentially one ester-forming alkyl group from the β-aminoglutaric acid di-alkyl ester without cleavage of the other ester-forming alkyl group.

The process according to the second aspect invention where $\beta$-aminoglutaric acid dimethyl ester is employed as the starting material may schematically be described by the following equation:—

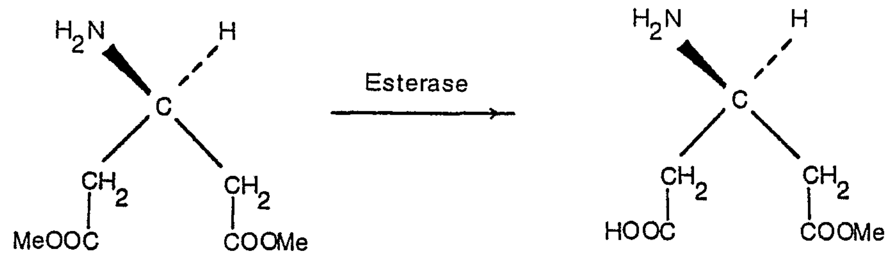


(VIII′) (VII′)

wherein Me denotes methyl group.

With the above equation, it is again seen that the $\beta$-aminoglutaric acid dimethyl ester (VIII′) is an optically inactive compound which is belonging to the compound of the "Cs-point" class having a symmetrical plane in the molecule, that this compound contains two methoxycarbonylmethyl groups which are positioning with each other in an enantropic relationship in the molecule thereof, and that only one of the two methoxycarbonylmethyl groups preferentially can undergo the enzymatic hydrolysis and bring about the removal of the ester-forming methyl group therefrom, affording the optically active (R)-aminoglutaric acid methyl ester (VII′). This reaction equation is stereochemically characterized in that the starting "prochiral" compound (VIII′) is converted into the "chiral" compound (VII′) by the above enzymatic hydrolysis with the esterase.

In the above-mentioned research of Cohen et al where the optically inactive $\beta$-acetylaminoglutaric acid di-alkyl ester is converted into the optically active $\beta$-(R)-acetylaminoglutaric acid mono-alkyl ester by the enzymatic hydrolysis with $\alpha$-chymotrypsin, it was found that very large amount of the enzyme was needed even with a long reaction time required.

As a result of our studies, we have now found that even if the 3-aminoglutaric acid di-alkyl ester which was considered to have the above-mentioned disadvantageous behaviour upon the enzymatic hydrolysis with $\alpha$-chymotrypsin is used as the substrate compound to be treated, this optically inactive compound can be converted into an optically active $\beta$-aminoglutaric acid mono-alkyl ester having the (R)-configuration without its amino group being protected, when the enzyme for use in the hydrolysis thereof is appropriately selected by careful investigation in accordance with the second aspect of this invention. Thus, we have succeeded to arrive at the new process of the second aspect of this invention as stated before.

The ester-hydrolyzing enzyme (esterase) used in the process of the second aspect of this invention may also be chosen from those of pig liver similarly to the first aspect of this invention.

The process according to the second aspect of this invention may be carried out in the same manner as in the process of the first aspect of this invention. Particularly, the reaction solvent, reaction pH, reaction temperature, reaction time and amount of the enzyme employed in the process of the second aspect invention are same as those employed according to the process of the first aspect invention.

After the enzymatic reaction is completed, the reaction solution is adjusted to a pH of not higher than 7.0 by addition of diluted hydrochloric acid and the desired product is extracted therefrom with methanol. The methanolic extract is concentrated to give a crude oily product which may be isolated and purified by cellulose column chromatography developed with n-butanol as the development solvent affording a product rich in a $\beta$-(R)-aminoglutaric acid mono-alkyl ester (about 40—50% enantiomeric excess) (VII).

The $\beta$-(R)-aminoglutaric acid mono-alkyl ester so produced has an optical purity which varies depending on the reaction conditions employed but is of a positive $[\alpha]_D$ in a range of from +2.36° to +4.72° (c 4.23, $H_2O$). The absolute stereochemistry of this product was determined to be R-configuration by cyclising this product into a $\beta$-lactam compound and then comparing the latter with an authentic sample of (S)-4-[(methoxycarbonyl)methyl]-2-azetidinone. Thus with the process of the second aspect of this invention, it is again observed that only one of the enantiomeric two alkoxycarbonylmethyl groups present in the starting substrate compound (VIII) preferentially undergoes the hydrolytic removal of the alkyl group of the "pro-S" nature therefrom, giving the $\beta$-(R)-aminoglutaric acid mono-alkyl ester (VII) preferentially.

$\beta$-(R)-Aminoglutaric acid mono-methyl ester and mono-ethyl ester as obtained by the second aspect invention are new compounds which exhibit an antibacterial activity against Gram-positive bacteria, particularly against *Staphylococcus aureus* and hence are useful as antibacterial agent.

Besides, this β-(R)-aminoglutaric acid mono-alkyl ester is readily convertible into an (R)-4-alkoxycarbonylmethylazetidine-2-on.

In consequence, the processes according to the first and second aspects of this invention are generically applicable as an enzyme-chemical process for preparing optically active β-amino acid intermediates in the free acid form of which the center of asymmetry can be selected by option according to the desired configuration of the asymmetrical carbon atom of the ultimately desired physiologically active substances, when said optically active β-amino acid is subjected to appropriate chemical conversions at the sites of its free amino group, free carboxylic group and/or ester group.

This invention is illustrated below with reference to Examples 1—2 (according to the first aspect of this invention) and Examples 3—6 (according to the second aspect of this invention).

Example 1

(a) A solution of β-benzyloxycarbonylaminoglutaric acid dimethyl ester (465 mg, 1.5 milimol) in acetone (1.5 ml) was admixed with 45 ml of a phosphate buffer solution (pH 8.0). The solution obtained was admixed with 2.7 mg (measured as the quantity of protein) of pig liver esterase (Esterase E—3128, a product of Shigma Co., U.S.A.). The resultant admixture was allowed to stand at 25°C for 6 hours under occasional stirring, and the resulting reaction solution was adjusted to pH 1 by addition of 2.5 ml of 2N hydrochloric acid thereto, followed by extraction with 50 ml of methylene chloride. The organic layer was dried over anhydrous sodium sulfate and concentrated to give 419 mg of a crude product as crystals. This crude product was purified by chromatography on silica gel developed with ethyl ether to afford 410 mg (93%) of optically active β-(S)-benzyloxycarbonylaminoglutaric acid mono-methyl ester which showed a specific optical rotation $[\alpha]_D^{25} = +0.71°$ (c 7.45, $CHCl_3$). mp. 97.0—97.5°C.

IR. spectrum (KBr): 3330, 2840—2960, 1740, 1725, 1700 $cm^{-1}$.

H—NMR. spectrum ($CDCl_3$): δ2.69 (bd, 4H, $CH_2$ J=6 Hz), δ3.65 (s, 3H, $CO_2Me$), δ4.17—4.53 (m, 1H, CH), δ5.07 (s, 2H, $CH_2Ph$), δ5.63 (bd, 1H, NH), δ7.30 (s, 5H, Ph).

Elemental analysis:

| | | | |
|---|---|---|---|
| Calcd. for $C_{14}H_{17}O_6N$: | C 56.94, | H 5.80, | N 4.74% |
| Found: | C 57.05, | N 5.84, | N 4.69% |

The β-(S)-benzyloxycarbonylaminoglutaric acid mono-methyl ester obtained as above is a new compound which is not described in any chemical literature.

(b) The mono-methyl ester (385 mg, 1.3 milimol) obtained as above was dissolved in 20 ml of methanol, and the resulting methanolic solution was admixed with 40 mg of 10% palladium-carbon and then stirred at ambient temperature for 30 minutes under atmosphere of hydrogen. The reaction solution was filtered and thefiltrate solution was concentrated to give 205 mg (98%) of β-(S)-aminoglutaric acid mono-methyl ester as oil which showed a specific optical rotation $[\alpha]_D^{25} = -5.52°$ (c 3.26, $H_2O$).

This product showed the following IR. and NMR. spectra and elemental analysis.

IR. spectrum 3440, 1730, 2400—2900 $cm^{-1}$.

$^1H$—NMR. spectrum ($D_2O$): δ2.59 (bd, 2H, J=6 Hz), δ2.84 (bd, 2H, J=6 Hz), δ3.76 (s, 3H, $CO_2CH_3$), δ3.85—4.05 (m, 1H, CH).

Elemental analysis:

| | | | |
|---|---|---|---|
| Calcd. for $C_6H_{11}O_4N$: | C 44.71, | H 6.88, | N 8.69% |
| Found: | C 44.73, | N 6.87, | N 8.65% |

The product of this Example 1 (b) was tested for its antibacterial activity and its minimum concentration inhibitory to the growth of *Staphylococcus aureus* was found to be 10—100 mcg/ml.

Example 2

The procedure (a) of Example 1 as above was repeated using 500 mg of β-benzhydrylaminoglutaric acid di-ethyl ester in stead of the β-benzyloxycarbonylaminoglutaric acid dimethyl ester. The product from the enzymatic hydrolysis was subjected to catalytic hydrogenolysis over palladiumcarbon in the same manner as in the procedure (b) of Example 1. β-(S)-Aminoglutaric acid mono-ethyl ester was obtained as oil in the yield of 400 mg.

IR. spectrum (KBr): 3445, 1728 $cm^1$.

This mono-ethyl ester also showed a potency value of 20—100 mcg/ml for its minimum concentration inhibitory to the growth of *Staphylococcus aureus*.

Example 3

(a) A solution of dimethyl β-t-butoxycarbonylaminoglutarate (413 mg, 1.5 milimol) in acetone (1.5 ml) was admixed with 45 ml of a phosphate buffer solution (pH 8.0). The solution obtained was

admixed with 2.7 mg (measured as the quantity of protein) of pig liver esterase (Esterase E—3128, a product of Shigma Co., U.S.A.). The resulting mixture was allowed to stand at 25°C for 6 hours under occasional stirring, and the reaction mixture was adjusted to pH 1 with hydrochloric acid thereto, followed by extraction with 50 ml of methylene chloride. The organic layer was dried over anhydrous sodium sulfate and concentrated to give 363 mg of a crude product as crystals. This crude product was purified by chromatography on silica gel developed with 2% methanol-methylene chloride to afford 320 mg of optically active β-(S)-t-butoxycarbonylaminoglutaric acid mono-methyl ester, showing mp. 94.1—97.0°C after recrystallization from ether-hexane.

IR. spectrum (KBr): 3330, 1735, 1720 $cm^{-1}$.

H—NMR. spectrum ($CDCl_3$): δ1.44 (s, 9H), δ2.67 (d, J=6, 4H), δ3.72 (s, 3H), δ4.38 (m, 1H).

Mass spectrum: 262 ($M^+$ + 1).

Elemental analysis:

| | | | |
|---|---|---|---|
| Calcd. for $C_{11}H_{19}NO_6$: | N 5.36, | C 50.56, | H 7.33% |
| Found: | N 5.31, | C 50.32, | H 7.43% |

The half ester, β-(S)-t-butoxycarbonylaminoglutaric acid mono-methyl ester obtained here is a new compound which is not described in any chemical literature.

Boc-aminoglutaric acid mono-methyl ester (100 mg) was treated with trifluoroacetic acid (1 ml) for 15 minutes and the mixture was dried under a reduced pressure. The residue was dissolved in water and neutralized with potassium carbonate, affording 80 mg of β-(S)-aminoglutaric acid mono-methyl ester after usual workup.

Example 4

A solution of β-aminoglutaric acid dimethyl ester (346 mg, 2.0 milimol) in 7 ml of a phosphate buffer solution (pH 8.0) was admixed with 2.89 mg (400 units) of pig liver esterase (Esterase E—3128, a product of Shigma Co., U.S.A.). The resultant admixture was allowed to stand for 25°C for 2.5 hours under occasional stirring, and the reaction solution was adjusted to a pH of about 7.0 by addition of 0.1 N hydrochloric acid, followed by concentration to dryness at a temperature of not higher than 20°C. The oily residue was extracted with 50 ml of methanol, and the methanolic extract was concentrated to give 268 mg of a crude product. This crude product was isolated and purified by chromatography on cellulose column (Cellulose CF—11, a product of Wattman Co., U.S.A.) developed with n-butanol saturated with water as the eluent, to afford 212 mg (66% yield) of β-(R)-aminoglutaric acid mono-methyl ester as oil which showed a specific optical rotation $[\alpha]_D^{22}$ +2.36° (c 4.23, $H_2O$).

IR spectrum (KBr): 3440, 1730, 2400—2900 $cm^{-1}$.

$^1$H—NMR. ($D_2O$): δ2.59 (bd, 2H, J=6 Hz), δ2.84 (bd, 2H, J=6 Hz), δ3.76 (s, 3H, $COOCH_3$), δ3.85—4.05 (m, 1H, CH).

Elemental analysis:

| | | | |
|---|---|---|---|
| Calcd. for $C_6H_{11}O_4N$: | C 44.71, | H 6.88, | N 8.69% |
| Found: | C 44.70, | N 6.86, | N 8.42% |

This new compound was tested for its antibacterial activity and its minimum concentration inhibitory to growth of *Staphylococcus aureus* was found to be 20 mcg/ml.

Example 5

The process of the Example 4 was repeated using 5.78 mg (400 units) of the pig liver esterase (Esterase E—3128). The methanolic extract obtained was concentrated to give 270 mg of a crude product, which was then purified by the cellulose column chromatography in the same way as in the Example 4 to afford 242 mg of β-(R)-aminoglutaric acid mono-methyl ester as oil which showed a specific optical rotation $[\alpha]_D^{22}$ +3.63° (c 4.23, $H_2O$).

Example 6

The process of the Example 4 was repeated using 10 mg (400 units) of the pig liver esterase (Esterase E—3128) and effecting the enzymatic reaction at 20°C for 1.5 hours under occasional stirring. The methanolic extract obtained was concentrated to give 280 mg of a crude product, which was then purified by the cellulose column chromatography in the same manner as in the Example 4 to afford 250 mg of β-(R)-aminoglutaric acid mono-methyl ester as oil which showed a specific optical rotation $[\alpha]_D^{22}$ +4.72° (c 4.23, $H_2O$).

Example 7

The process of the Example 6 was repeated except that 400 mg of β-aminoglutaric acid diethyl ester was used instead of the dimethyl ester.

$\beta$-(R)-Aminoglutaric acid mono-ethyl ester was afforded in a yield of 320 mg as oil which showed a specific optical rotation $[\alpha]_D^{22}$ +3.84° (c 4.20, $H_2O$).

This new compound was tested for its antibacterial activity, and found its minimum concentration inhibitory to growth of *Staphylococcus aureus* was found to be 30—100 mcg/ml.

**Claims**

1. A process for the production of an optically active $\beta$-(S)-aminoglutaric acid mono-alkyl ester of the formula

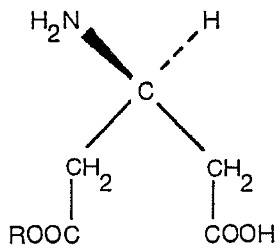

(IV)

wherein R is an alkyl group of 1 to 6 carbon atoms, which comprises the steps of:—

reacting a $\beta$-N-protected aminoglutaric acid di-alkyl ester of the formula

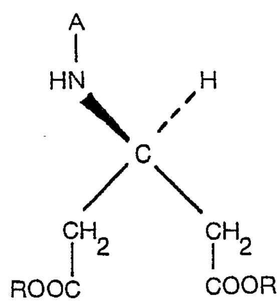

(V)

wherein R is as defined above and A is an amino-protecting group removable by hydrogenolysis or mild hydrolysis, with pig liver esterase in an organic solvent at a pH of 7.0 to 9.0 and at a temperature of 20—30°C, to remove hydrolytically and preferentially one ester-forming alkyl group (R) from the $\beta$-N-protected aminoglutaric acid di-alkyl ester without cleavage of the other ester-forming alkyl group,

and then removing the residual amino-protecting group (A) from the resulting hydrolysis product in a known manner.

2. The process of Claim 1 in which the $\beta$-N-protected aminoglutaric acid di-alkyl ester is $\beta$-N-benzyloxycarbonylaminoglutaric acid dimethyl or diethyl ester.

3. A process for the production of an optionally active $\beta$-(R)-aminoglutaric acid mono-alkyl ester of the formula

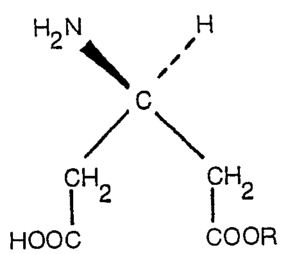

(VII)

wherein R is an alkyl group of 1 to 6 carbon atoms, which comprises the steps of:—

reacting a $\beta$-aminoglutaric acid di-alkyl ester of the formula

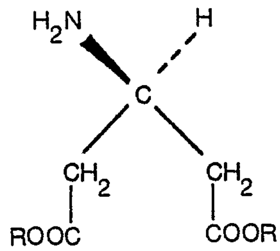

(VIII)

wherein R is as defined above, with pig liver esterase in an organic solvent at a pH of 7.0—9.0 and at a temperature of 20 to 30°C, to remove hydrolytically and preferentially one ester-forming alkyl group from the β-aminoglutaric acid di-alkyl ester without cleavage of the other ester-forming alkyl group.

4. The new compound which is β-(S)-benzyloxycarbonylaminoglutaric acid monomethyl ester; β-(S)-aminoglutaric acid monomethyl ester; and β-(S)-aminoglutaric acid monoethyl ester.

5. The new compound which is β-(R)-aminoglutaric acid monomethyl ester and β-(R)-aminoglutaric acid mono-ethyl ester.

**Patentansprüche**

1. Verfahren zur Herstellung von optisch aktiven β-(S)-Aminoglutarsäure-monoalkylestern der Formel

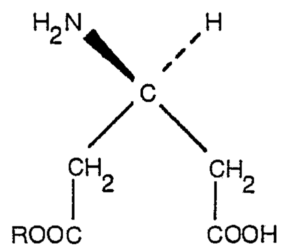

(IV)

worin R eine Alkylgruppe mit 1 bis 6 Kohlenstoffatomen darstellt, gekennzeichnet durch folgende Stufen:
Umsetzung eines β-N-geschützten Aminoglutarsäure-dialkylesters der Formel

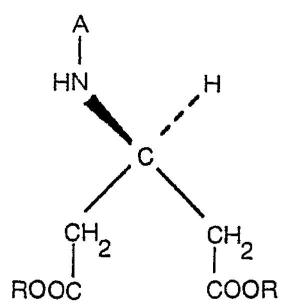

(V)

worin R die oben angegebene Bedeutung hat und A für eine durch Hydrogenolyse oder milde Hydrolyse abspaltbare Aminoschutzgruppe steht, mit Schweineleberesterase in einem organischen Lösungsmittel bei pH 7,0 mit 9,0 und einer Temperatur von 20—30°C, um

hydrolitisch und vorzugsweise eine esterbildende Alkyl gruppe (R) aus dem β-N-geschützten Aminoglutarsäure-dialkylester ohne Spaltung der anderen esterbildenden Alkylgruppe zu entfernen, und anschliessende Entfernung der verbliebenen Aminoschutzgruppe (A) aus dem entstandenen Hydrolyseprodukt in bekannter Weise.

2. Verfahren nach Anspruch 1, worin als β-N-geschützer Aminoglutarsäure-dialkylester β-N-Benzyloxycarbonylaminoglutarsäure-dimethyl- oder -diäthylester vorliegt.

3. Verfahren zur Herstellung optisch aktiver β-(R)-Aminoglutarsäure-monoalkylester der Formel

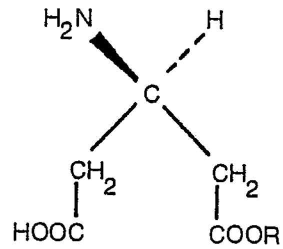

(VII)

worin R eine Alkylgruppe mit 1 bis 6 Kohlenstoffatomen darstellt, gekennzeichnet durch folgende Stufen:
Umsetzung eines $\beta$-Aminoglutarsäure-dialkylesters der Formel

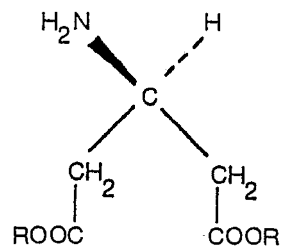

(VIII)

worin R die oben angegebene Bedeutung hat, mit Schweineleberesterase in einem organischen Lösungsmittel bei pH 7,0—9,0 und einer Temperatur von 20 bis 30°C, um hydrolytisch und vorzugsweise eine esterbildende Alkylgruppe aus dem $\beta$-Aminoglutarsäure-dialkylester ohne Spaltung der anderen esterbildenden Alkylgruppe zu entfernen.

4. $\beta$-(S)-Benzyloxycarbonylaminoglutarsäure-monoethylester, $\beta$-(S)-Aminoglutarsäure-monomethylester und $\beta$-(S)-Aminoglutarsäure-monoäthylester als neue Verbindungen.

5. $\beta$-(R)-Aminoglutarsäure-monomethylester und $\beta$-(R)-Aminoglutarsäure-monoäthylester als neue Verbindungen.

## Revendications

1. Procédé pour la production d'un mono-ester alcoylique de l'acide $\beta$-(S)-aminoglutarique optiquement actif, répondant à la formule:

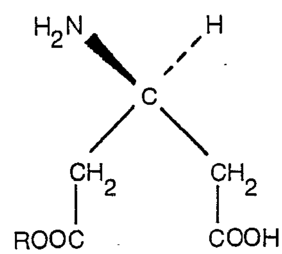

(IV)

dans laquelle R est un groupe alcoyle ayant de 1 à 6 atomes de carbone, procédé caractérisé en ce qu'il comporte les étapes consistant à

faire réagir un di-ester alcoylique de l'acide $\beta$-aminoglutarique N-protégé de formule

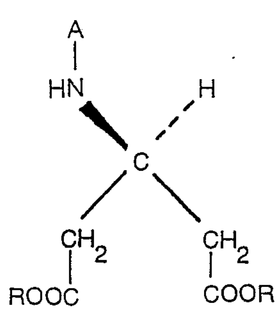

(V)

dans laquelle R est tel que défini ci-dessus et A est un groupe amino-protecteur éliminable par hydrogénolyse ou hydrolyse modérée, avec l'estérase de foie de porc, dans un solvant organique à un pH de 7,0 à 9,0 et à une température de 20 à 30°C pour éliminer par hydrolyse et de manière préférentielle un groupe alcoyle (R) estérifiant du di-ester alcoylique de l'acide β-aminoglutarique N-protégé sans séparer l'autre groupe alcoyle estérifiant,

et à éliminer ensuite le groupe amino-protecteur résiduel (A) du produit d'hydrolyse résultant, d'une manière connue.

2. Procédé selon la revendication 1, caractérisé en ce que le di-ester alcoylique de l'acide β-aminoglutarique N-protégé est l'ester diméthylique ou diéthylique de l'acide β-N-benzyloxycarbonyl-aminoglutarique.

3. Procédé pour la production d'un mono-ester alcoylique de l'acide β-(R)-aminoglutarique optiquement actif répondant à la formule

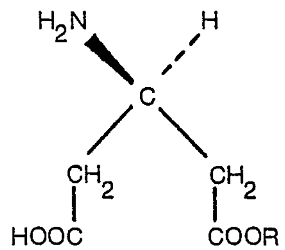

(VII)

dans laquelle R est un groupe alcoyle ayant de 1 à 6 atomes de carbone, procédé caractérisé en ce qu'il comporte les étapes consistant à

faire réagir un di-ester alcoylique de l'acide β-aminoglutarique de formule

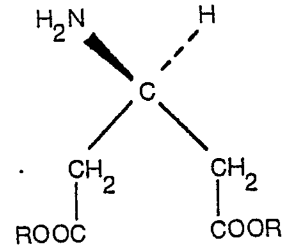

(VIII)

dans laquelle R est tel que défini ci-dessus, avec l'estérase de foie de porc, dans un solvant organique à un pH de 7,0 à 9,0 et à une température de 20 à 30°C pour éliminer par hydrolyse et de manière préférentielle un groupe alcoyle estérifiant du di-ester alcoylique de l'acide β-aminoglutarique, sans séparer l'autre groupe alcoyle estérifiant.

4. Nouveau composé caractérisé en ce qu'il est l'ester monométhylique de l'acide β-(S)-benzyloxycarbonylaminoglutarique; l'ester monométhylique de l'acide β-(S)-aminoglutarique; et l'ester mono-éthylique de l'acide β-(S)-aminoglutarique.

5. Nouveau composé caractérisé en ce qu'il est l'ester monométhylique de l'acide β-(R)-aminoglutarique et l'ester mono-éthylique de l'acide β-(R)-aminoglutarique.